# EUROPÄISCHE PATENTSCHRIFT

(11) **EP 1 745 855 B1**
(45) Veröffentlichungstag und Bekanntmachung des Hinweises auf die Patenterteilung: **29.07.2009**
(21) Anmeldenummer: 06023416.8
(22) Anmeldetag: 14.09.2000
(51) Int. Cl.: B05B 11/00, A61M 15/00, B05B 11/02, A61M 5/315

(54) **Spender zum ggf. zerstäubten Ausbringen eines insbesondere flüssigen Mediums aus einem Behältnis**
Dispenser for dispensing, in particular spraying, a fluid from a container
Distributeur, notamment pour pulvériser un produit fluide contenu dans un réservoir

(30) Priorität: 15.09.1999 DE 19944209
(43) Veröffentlichungstag der Anmeldung: 24.01.2007
(62) Teilanmeldung aus: 00119964.5
(73) Patentinhaber: Ing. Erich Pfeiffer GmbH, 78315 Radolfzell (DE)
(72) Erfinder: Greiner-Perth, Jürgen, 78244 Gottmadingen (DE)
(74) Vertreter: Patentanwälte Ruff, Wilhelm, Beier, Dauster & Partner

(56) Entgegenhaltungen:
- EP-A- 0 829 307
- WO-A-98/30335
- DE-A- 4 008 068
- DE-A1- 19 749 514
- US-A- 4 962 868

## Beschreibung

Die Erfindung betrifft Spender zum ggf. zerstäubten Ausbringen eines insbesondere flüssigen Mediums aus einem Behältnis.

Ein derartiger Spender ist beispielsweise aus der EP 0 334 349 A1 bekannt, bei der das Ausbringen des Mediums durch manuelle Betätigung eines Betätigungselements erfolgt. Dabei ist die Betätigung in eine Folge von Teilbetätigungen untergliedert, wobei bei jeder Teilbetätigung eine definierte Teilcharge des Mediums ausgebracht wird. Um zu verhindern, dass zwei aufeinander folgende Teilbetätigungen ununterbrochen hintereinander durchgeführt werden, ist es aus dieser Druckschrift auch bekannt, dass zwischen zwei Teilbetätigungen des Betätigungselementes eine Umschaltbetätigung erforderlich ist.

Die DE 40 08 068 A1 beschreibt einen Zweimal-Spender nach dem Oberbegriff des Anspruchs 1. Seine Kulissenbahnen wirken mit einem Gleitstein so zusammen, dass dieser nach einer Teilbetätigung durch eine axiale Rückbewegung von einem Bahnabschnitt in den nächsten geführt wird.

Aus der EP 0 951 362 B1 ist ein Zweimal-Spender bekannt, bei dem eine manuelle Umschaltung für zwischen der ersten und folgenden Teilbetätigung erfolgt ist, wobei zerstörbare Druckpunktsicherungen vorgesehen sind.

Derartige Spender finden insbesondere Anwendung, wenn ein Medikament oder ein Impfstoff auf die Nasenschleimhäute eines Patienten appliziert werden soll. Ein zerstäubtes Ausbringen des Mediums hat dabei den Vorteil, dass die Absorption des Wirkstoffs durch den Patienten leichter erfolgt. Ein Beispiel, bei dem der Spender auch geeignet sein soll, durch den Patienten selbst genutzt zu werden, sind auf die Nasenschleimhäute zu applizierende Migränemittel.

Das Medium muss dabei in üblicherweise zwei, jedenfalls aber in einer geringen Anzahl, von Teilchargen definierter - meist gleicher - Größe aus dem Spender ausgebracht werden. Im Interesse einer großen Benutzungssicherheit wird dabei vorgeschlagen, den Betätigungsweg des Betätigungselements bewusst zu unterbrechen, damit nicht versehentlich in einer durchgehenden Betätigung des Betätigungselements mehr als eine Teilcharge auf einmal ausgebracht wird. Um dies sicherzustellen, ist zwischen den Teilbetätigungen des Betätigungselements eine Umschaltbetätigung erforderlich.

Die Durchführung einer solchen Umschaltbetätigung erfordert jedoch ein Absetzen des Spenders vom Applikationsort und steht einer Einhandbetätigung des Spenders entgegen.

Eine Einhandbetätigung eines solchen Spenders ist jedoch insbesondere dann wünschenswert, wenn der Benutzer die zweite Hand zur Fixierung des Ausbringungsortes - beispielsweise zur Fixierung des Kopfes eines Patienten, insbesondere eines Kindes - benötigt, die somit nicht zur Betätigung des Spenders selbst zur Verfügung steht. Ein Absetzen des Spenders und ein Lösen der Fixierung des Applikationsortes zur Durchführung der Umschaltbetätigung und eine anschließende Neufixierung des Applikationsortes und ein neues Ansetzen des Spenders wäre sehr umständlich.

### AUFGABE UND LÖSUNG

Aufgabe der Erfindung ist es daher, einen derartigen Spender dahingehend weiterzubilden, dass eine Einhandbetätigung, bei der sichergestellt ist, dass ein Ausbringen von mehr als einer Teilcharge in einer Betätigung zuverlässig verhindert ist, ermöglicht wird, jedoch für die Betätigung ausreichende Ausbringqualität sichergestellt ist.

Diese Aufgabe wird bei Zugrundelegen eines gattungsgemäßen Spenders erfindungsgemäß durch die kennzeichnenden Merkmale des Anspruchs 1 gelöst.

Bei einem erfindungsgemäßen Spender zum ggf. zerstäubten Ausbringen eines insbesondere flüssigen Mediums aus einem Behältnis erfolgt die Betätigung des Spenders in einer Folge von manuellen Teilbetätigungen eines Betätigungselements. Bei jeder der Teilbetätigungen des Betätigungselements wird aus dem Spender eine definierte Teilcharge ausgebracht. Zwischen zwei Teilbetätigungen des Betätigungselements erfolgt dabei eine Umschaltbetätigung. Diese Umschaltbetätigung erfolgt nach Durchführung einer Teilbetätigung jeweils selbsttätig.

Gemäß vorteilhaften Ausgestaltungen der Erfindung sind zur Durchführung der Umschaltbetätigung Kraftspeicher vorgesehen, die während der Durchführung einer Teilbetätigung vorgespannt werden. Diese Kraftspeicher können gleichzeitig auch dazu dienen, das Betätigungselement aus einer Betätigungsendlage, die erreicht wird, wenn eine Teilbetätigung vollständig durchgeführt wurde, in die Betätigungsausgangslage zurückzuführen, wobei aus der Betätigungsausgangslage heraus die nachfolgende Teilbetätigung durchgeführt werden kann. Es ist insbesondere vorteilhaft, die Umschaltbetätigung dann durchzuführen, wenn das Ende der Teilbetätigung dadurch festgestellt wird, dass die Betätigungskraft die zur Durchführung der Teilbetätigung erforderlich ist, nicht mehr auf das Betätigungselement einwirkt. Als Kraftspeicher können gemäß Ausgestaltung der Erfindung Federn, insbesondere Spiralfedern verwendet werden.

Gemäß weiteren Ausgestaltungen der Erfindung ist das Behältnis in einem Gehäuse angeordnet, wobei zwischen Gehäuse und Betätigungselement während jeder Teilbetätigung eine Relativbewegung stattfindet, wobei diese Relativbewegung durch die Kulissenführung festgelegt ist. Die Kulissenführung erfolgt insbesondere durch eine Kulissenbahn, in der ein Gleitstein geführt ist. Gemäß einer bevorzugten Ausführungsform ist zwischen Gehäuse und Behältnis eine Hülse angeordnet, wobei die Hülse entweder zum Gehäuse oder zum Behältnis lagefest bzgl. einer Teilbetätigung des Betätigungselements jedoch in Richtung der Umschaltbetätigung relativ beweglich hierzu angeordnet ist. Die Hülse weist dabei entweder die Kulissenbahn oder aber den in der Kulissenbahn geführten Gleitstein auf. Weitere bevorzugte Ausbildungen der Kulissenführung sind in den weiteren Unteransprüchen aufgeführt. Diese betreffen insbesondere die Gestaltung der Kulissenbahn selbst sowie die Ausbildung von Kraftspeichern an der Kulissenbahn.

Neben den Unteransprüchen ist die Erfindung anhand der in der Zeichnung dargestellten Ausführungsbeispiele näher erläutert; dabei zeigt:
- Fig. 1: ein Schnittbild durch einen ersten erfindungsgemäßen Spender, bei dem eine Hülse mit Kulissenbahn lagefest zu einem das Behältnis aufnehmenden Gehäuse angeordnet ist;
- Fig. 2a, 2b: Seitenansichten einer Hülse zur Verwendung in der Ausführungsform gemäß Fig. 1;
- Fig. 3a, 3b bis Fig. 6a, 6b: jeweils in Querschnittsdarstellung und in teilgeschnittener Darstellung Ansichten eines zweiten Ausführungsbeispiels, bei dem die Hülse lagefest zum Behältnis angeordnet ist, während unterschiedlicher Stellung, wie sie sich im Ablauf einer Folge von Teilbetätigungen des Spenders ergeben.

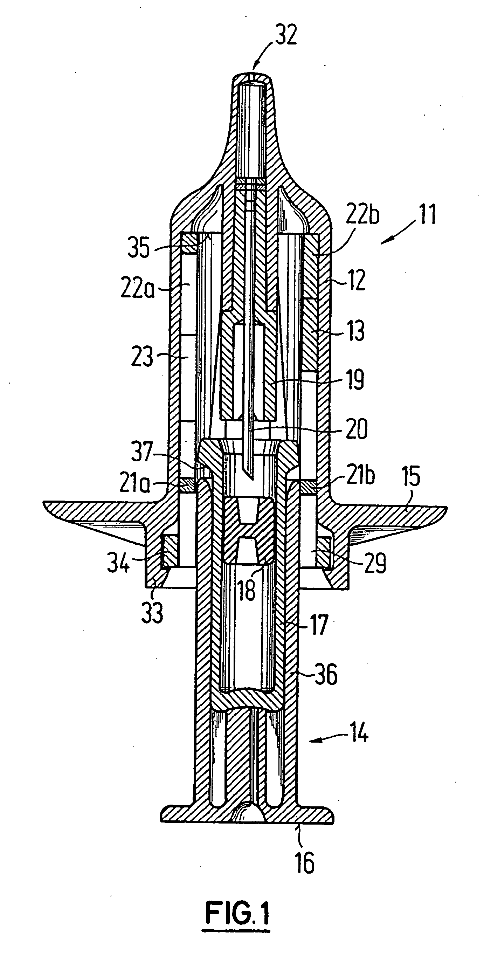

### BESCHREIBUNG DES AUSFÜHRUNGSBEISPIELS GEMÄSS FIG. 1 BIS FIG. 2b

Die Fig. 1 zeigt in Querschnittsdarstellung einen erfindungsgemäßen Spender, der in Schubkolbenbauweise ausgeführt ist. Der Spender 11 besteht aus einem Gehäuse 12, der im Gehäuse drehbar gehaltenen Hülse 13, dem Betätigungselement 14, das zugleich als Aufnahme für die Ampulle 17, die als Behältnis für das auszubringende Medium dient, besteht. Die Ampulle 17 ist vor der ersten Betätigung durch den Stopfen 18 verschlossen. Die Ampulle 17 ist in dem Betätigungselement 14 so gehalten, dass sie bei einer Betätigung des Betätigungselements in Richtung auf das Gehäuse hin mit dem Betätigungselement 14 mitbewegt wird. Während des ersten Betätigungsteilhubes wird zunächst der Stopfen 18 von der Kanüle 20, die einen Ausbringungskanal bis zu der Düse 32 bildet, durchstochen. Anschließend wird der Stopfen 18, der dann als Kolben wirkt, von der im Gehäuse 12 angeordneten Kolbenstange 19 in die Ampulle hineingedrückt, so dass das für das Medium zur Verfügung stehende Volumen der Ampulle verringert wird. Das Medium entweicht über die Kanüle 20 und die Düse 32 und wird so ausgebracht. Aufgrund der Unterteilung des Betätigungsweges, im vorliegenden in Fall zwei Teilbetätigungen, wird der Stopfen 18 bei jeder der beiden Teilbetätigungen nur um ein vorgegebenes Maß vorliegend also nur um die Hälfte des gesamten möglichen Betätigungsweges in die Ampulle hineingedrückt.

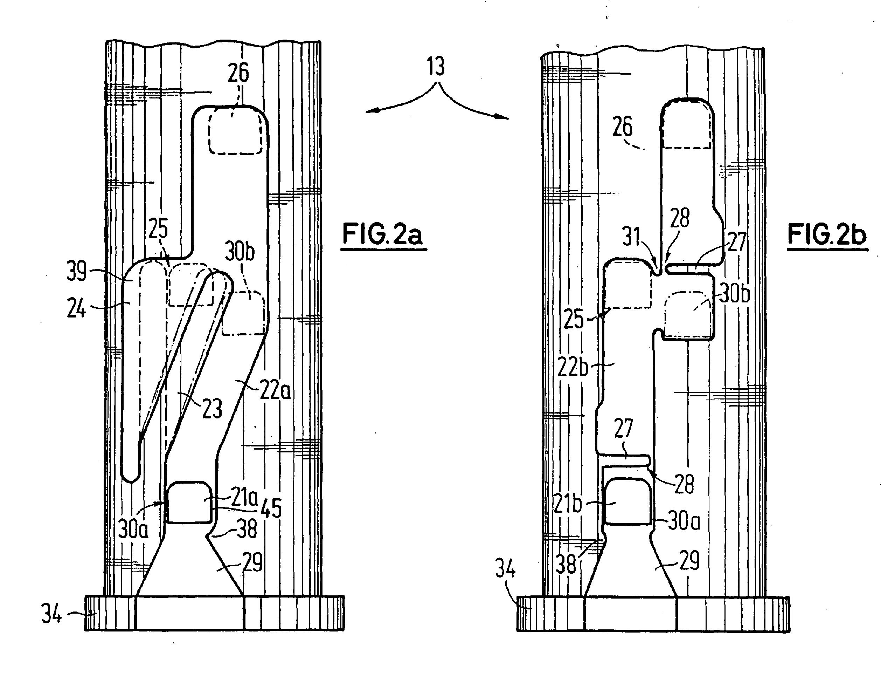

Das Gehäuse 12 des Spenders 11 weist an seinem vorderen Ende die Düse 32 auf, an seinem hinteren Ende die Fingerauflage 15 zur Auflage wenigstens eines Fingers. Die Fingerauflage 15 dient zur Erbringung einer die auf das Betätigungselement 14 einwirkende Betätigungskraft abstützenden Kraft auf das Gehäuse, so dass der Spender während der Betätigung ruhig gehalten ist. Im Gehäuse ist entlang der Gehäusewandung die Hülse 13 angeordnet. Die Hülse 13 stützt sich in der axialen Betätigungsrichtung des Betätigungselements 14 an der Stützkante 35 des Gehäuses 12 ab. An ihrem hinteren Ende weist die Hülse 13 einen Hülsenrand 34 auf, der von der gehäuseseitigen Rastkante 33 so hintergriffen wird, dass die Hülse 13 nicht nach hinten aus dem Gehäuse 12 herausgezogen werden kann. Durch Stützkante 35 und Rastkante 33 ist somit eine Lagerung der Hülse 13 im Gehäuse gebildet, die in Betätigungsrichtung des Betätigungselements 14 eine lagefeste Halterung der Hülse 13 im Gehäuse 12 definiert. Dabei kann die Hülse 13 im Gehäuse 12 um eine in Betätigungsrichtung des Betätigungselements 14 verlaufende Drehachse verdreht werden. Das Betätigungselement 14 weist an seinem hinteren Ende eine Betätigungsfläche 16 auf, die der manuellen Betätigung des Betätigungselements 14 insbesondere mittels des Daumens des Benutzers dient. Im übrigen wird das Betätigungselement 14 im wesentlichen aus dem hohlzylindrischen Schaft 36 gebildet. Dabei weist der hohlzylindrische Schaft 36 einen Außendurchmesser auf, der im wesentlichen dem Innendurchmesser der Hülse 13 entspricht. Der Schaft 36 wird im Gehäuse 12 durch die Hülse 13 geführt. Zur Ausbildung einer Kulissenführung weist die Hülse 13 auf einander gegenüberliegenden Seiten je eine Kulissenbahn auf, wie sie in der Fig. 2a, Fig. 2b näher dargestellt sind. Im Bereich des vorderen Endes des Schaftes 36 des Betätigungselements 14 sind an entsprechenden Stellen Gleitsteine 21a, 21b ausgebildet. In den Schaft 36 wird die Ampulle 17 mit dem darin bevorrateten Medium eingesetzt. Dabei entspricht der Innendurchmesser des Schaftes im wesentlichen dem Außendurchmesser der Ampulle, so dass eine klemmende Halterung der Ampulle 17 im Schaft 36 des Betätigungselements 14 erfolgt. Ferner weist die Ampulle an ihrem vorderen Ende eine Querschnittserweiterung auf, die als Stoßkante 37 in Anlage mit der vorderen Kante des Schaftes 36 gelangen kann, so dass sichergestellt ist, dass die Ampulle 17 bei Betätigung des Betätigungselements 14 in dessen Betätigungsrichtung spielfrei mitgeführt wird.

Die Fig. 2a, 2b zeigen gegenüberliegende Seitenansichten der Hülse 13 mit den Kulissenbahnen 22a, 22b und den jeweils in der Kulissenbahn geführten Gleitsteinen 21a, 21b. Dabei dient die in der Fig. 2a dargestellte Kulissenbahn 22a der Erzeugung der Vorspannung eines Kraftspeichers zur Durchführung der Umschaltbetätigung zwischen den beiden Teilbetätigungen des Betätigungselements während die in Fig. 2b gezeigte Kulissenbahn der Unterteilung des Betätigungsweges des Betätigungselements in zwei Teilbetätigungen dient.

Beide Kulissenbahnen weisen im Bereich des Hülsenrandes 34 einen Einführtrichter 29 zur einfachen Einführung der Gleitsteine 21a, 21b in die jeweilige Kulissenbahn 22a, 22b auf. Ferner sind jeweils Druckpunktmittel 38 im Endbereich der Einführtrichter 29 ausgebildet, die ein Herausgleiten der Gleitsteine 21a, 21b nach hinten aus der Kulissenbahn 22a bzw. 22b verhindern. Somit wird eine Verlustsicherung für das Betätigungselement 14 gebildet.

Die in Fig. 2a dargestellte Kulissenbahn weist einen Federarm 23 auf, der in den Bewegungsraum des Gleitsteins 21a hineinragt und von diesem bei der ersten Teilbetätigung unter Erzeugung einer Vorspannung in den, im Bereich der Hülse materialfreien Verdrängungsraum 39 hineingedrückt wird. Der Federarm 23 besteht vorzugsweise aus dem gleichen Material wie die Hülse selbst und kann als dünner Materialsteg in dem materialfreien Bereich der Kulissenbahn 22a ausgebildet sein. Vorzugsweise wird die Hülse 13 aus Kunststoff hergestellt, wobei beim Spritzgießen der Hülse 13 im selben Spritzvorgang gleich der Federarm 23 erzeugt werden kann.

Das Verdrängen des Federarms 23 aus dem Bewegungsraum des Gleitsteins 21a wird dadurch erforderlich, dass die Bahnkurve der in Fig. 2b dargestellten gegenüberliegend auf der Hülse angeordneten zweiten Kulissenbahn 22b über die gesamte Länge einer ersten Teilbetätigung hinweg geradlinig verläuft. Dadurch wird verhindert, dass der Gleitstein 21a geführt durch den Federarm 23 nicht schon jetzt eine Drehbewegung der Hülse 13 im Gehäuse 12 erzeugt.

Die Fig. 2a zeigt auch eine vorteilhafte Ausführungsform des Gleitsteins 21a. Er ist nur auf seiner Vorderseite verrundet um ein Verteilen in der Kulissenbahn 22a zu vermeiden. Er weist darüber hinaus wenigtens eine Flanke 45 auf, die aufgrund ihrer Längserstreckung parallel zu den linearen Bahnabschnitten der Kulissenbahn eine lange Führung des Gleitsteins 21a in der Kulissenbahn 22a sicherstellt. Auch der in der Fig. 2b dargestellte Gleitstein 21b kann eine entsprechende Form mit einer Flanke 45 aufweisen.

Wie aus der Fig. 2b ersichtlich, ist der Gleitstein 21b in einer Kulissenbahn 22b geführt, die aus zwei linearen Bahnabschnitten besteht, die lediglich um in etwa die Breite der Kulissenbahn zueinander versetzt sind, wobei die Länge der beiden geradlinigen Abschnitte jeweils dem Betätigungsweg einer Teilbetätigung des Betätigungselements 14 entspricht.

Dabei überlappen sich die beiden geradlinigen Abschnitte in einem Bereich dessen Länge in etwa dem Durchmesser des Gleitsteins 21b entspricht. Wird das Betätigungselement 14, an dem die Gleitsteine 21a, 21b betätigt, so läuft zunächst der Gleitstein 21b auf das Sperrmittel 27, einem dünnen, an einer Seite der Kulissenbahn 22b abragenden Materialsteg, der über eine Sollbruchstelle 28 mit der gegenüberliegenden Kante der Kulissenbahn 22b verbunden ist und nach Abtrennung an der Sollbruchstelle vollständig aus der Kulissenbahn herausführbar ist, auf. Erst wenn an dem Betätigungselement 14 eine Betätigungskraft aufgebracht wird, die zum Bruch an der Sollbruchstelle 28 führt, wird der das Sperrmittel 27 bildende Materialsteg aus der Kulissenbahn 22b seitlich herausgedrückt. Das Sperrmittel 27 mit seiner Sollbruchstelle 28 stellt somit sicher, dass am Betätigungselement 14 eine derartige Betätigungskraft aufgebracht ist, dass eine vollständige Teilbetätigung des Betätigungselementes in einem zusammenhängenden Betätigungsablauf durchgeführt wird. Der Gleitstein 21b gleitet dann in einer linear verlaufenden, ununterbrochenen Bewegung bis zu der am Ende des ersten linearen Abschnittes der Kulissenbahn 22b ausgebildeten ersten Teilbetätigungsendlage 25. In dieser Lage liegt der in Fig. 2a dargestellte Gleitstein 21a im Bereich des vorderen Endes des Federarms 23 der im dargestellten Beispiel nach links aus dem Bewegungsraum des Gleitsteins 21a herausgedrückt wurde und nun gegen diesen mit entsprechender Hebellänge und der aufgrund der Materialverbiegung erzeugten Kraft gegen den Gleitstein 21a drückt.

Wenn das Betätigungselement 14 selbst nicht zum Gehäuse 12 verdrehbar ist - hier kann die Hemmungswirkung, die durch die im Stopfen 18 befindliche Kanüle 20 sowie durch die am Stopfen und ggf. auch an der Innenseite der Ampulle anliegenden Kolbenstange 19 des Gehäuses 12 entsteht, zu groß sein - wird die Hülse 13 gegenüber dem Betätigungselement 14 mit den daran ausgebildeten Gleitsteinen 21a, 21b aufgrund der im Federarm 23 erzeugten Kraft verdreht. Dies ist nur im Überlappungsbereich der beiden geradlinigen Abschnitte der Kulissenbahn 22b möglich. Um eine saubere Trennung zwischen den beiden Teilbetätigungen zu erreichen, ist am der ersten Teilbetätigungs-Endlage 25 entsprechenden Bereich des ersten geradlinigen Abschnittes der Kulissenbahn 22b eine Entlastungsnase 31 ausgebildet, die ein Verdrehen der Hülse 13 bzgl. der Gleitsteine 21a, 21b so lange verhindert, wie nicht das Betätigungselement 14 vollständig von der Betätigungskraft entlastet wurde. Erst danach kann die Entlastungsnase überwunden werden, wobei dabei die Hülse in vernachlässigbarer Weise in Betätigungsrichtung des Betätigungselements 14 verschoben wird. Durch das Verdrehen der Hülse bzgl. den Gleitsteinen 21a, 21b und den das die Hülse 13 haltenden Gehäuses 12 erreicht der Gleitstein 21b die Betätigungsausgangslage 30b. Durch erneute Betätigung des Betätigungselements 14 gelangt der Gleitstein 21b zunächst wiederum in Anlage an Sperrmittel 27 mit einer Sollbruchstelle 28, die mit einer Mindestbetätigungskraft am Betätigungselement überwunden werden muss. Danach wird der Bewegungsraum für die zweite Teilbetätigung, gebildet durch den zweiten linearen Abschnitt der Bahnkurve der Kulissenbahn 22b freigegeben. Die Kulissenbahn 21a ist dabei, wie in Fig. 2a dargestellt, so ausgebildet, dass sowohl die Drehbewegung als auch die Teilbetätigungsbewegung durch den Gleitstein 21a innerhalb der Bahnkurve der Kulisse 22a freigegeben ist.

Eine das Zurückziehen des Betätigungselements 14 nach Abschluss der Teilbetätigungen verhindernde Rückführung wird durch die in der Fig. 2a dargestellte Bahnkurve der Kulissenbahn 22a in Zusammenwirken mit dem Federarm 23 und der Formgebung des Gleitsteins 21a dadurch erreicht, dass der Gleitstein beim Zurückziehen des Betätigungselements mittels Federarm 23 in den toten Schacht 24 hineinbewegt wird, sobald die Umschaltbetätigung durch das Federelement 23 durchgeführt wurde.

Alternativ ist es auch möglich, die Hülse 13 im Gehäuse 12 auch drehfest anzuordnen. Dann ist darauf zu achten, dass die Hemmung - insbesondere zwischen Stopfen 18 und Kanäle 20 - nicht so groß ist, dass ein Verdrehen der Ampulle 17 samt Betätigungselement 14 in der Hülse 13 aufgrund der durch den Federarm 23 erzeugten Kraft nicht verhindert wird. Eine solche Ausgestaltung hat den Vorteil, dass insbesondere bei einer beispielsweise durch Verrippung rauhen Betätigungsfläche 16, ein Verdrehen von Ampulle 17 und Betätigungselement 14 erst möglich ist, wenn der Benutzer das Betätigungselement loslässt. Das verhindert ebenfalls zuverlässig, dass zwei Teilbetätigungen ohne Unterbrechung hintereinander durchgeführt werden.

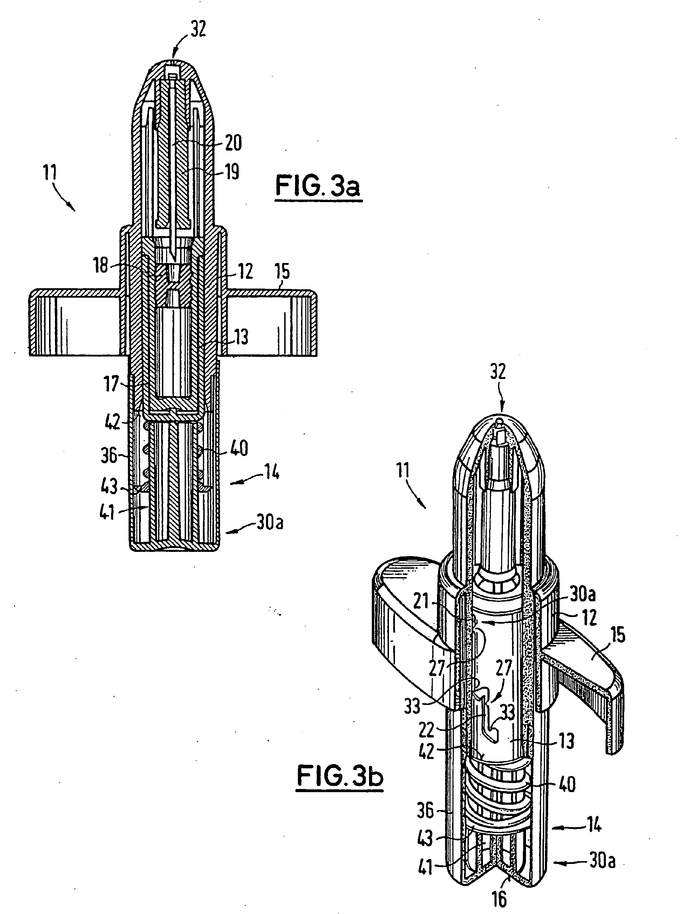

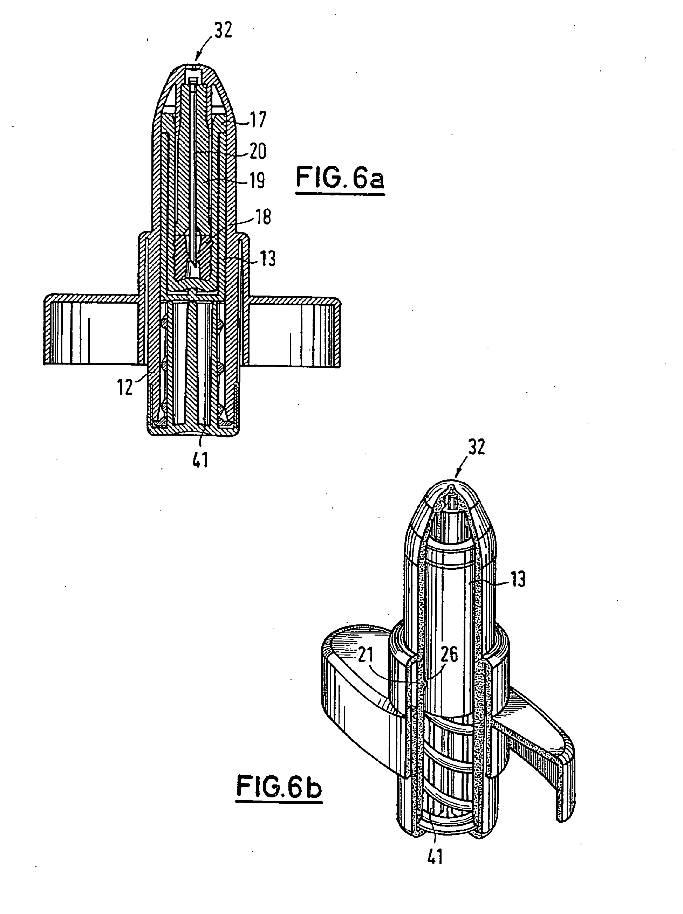

### BESCHREIBUNG DES AUSFÜHRUNGSBEISPIELS GEMÄSS DEN FIG. 3a, 3b BIS 6a, 6b

Die Figuren 3a, 3b bis Figuren 6a, 6b zeigen jeweils in Schnittdarstellung und in teilgeschnittener Perspektivdarstellung die unterschiedlichen Betätigungsstellungen eines erfindungsgemäßen Spenders, bei dem die Teilbetätigungen des Betätigungselements über eine Kulissenführung erzeugt wird, wobei die Kulissenführung eine Hülse nutzt, die in Betätigungsrichtung des Betätigungselements lagefest zum Behältnis für das auszubringende Medium angeordnet ist.

Die Figuren 3a, 3b zeigen die Darstellung vor der ersten Betätigung des Spenders 11. Der Spender 11 besteht aus einem Gehäuse 12, mit der an ihm ausgebildeten Fingerauflage 15. In dem Gehäuse 12 ist, in der Hülse 13 geführt, die Ampulle 17 gehalten. Dabei ist die Ampulle 17 durch den Stopfen 18 verschlossen. Das Medium wird dadurch aus der Ampulle 17 ausgebracht, dass bei der ersten Betätigung die Kanüle 20 den Stopfen 18 durchsticht und danach der Stopfen 18, als Kolben wirkend, synchron mit der Kanüle 20 relativ bewegt zur Ampulle 10 verschoben wird, dass das Medienvolumen in der Ampulle verringert wird. Dazu wird der Stopfen 18 von der Kolbenstange 19 beaufschlagt, sobald die Kanüle 20 den Stopfen 18 durchstochen hat. Das Medium selbst tritt dann durch den durch die Kanüle 20 gebildeten Ausbringungskanal aus der Ampulle aus und gelangt zur Düse 32 in dem Gehäuse 12, wo das Medium zerstäubt und aus dem Spender ausgebracht wird. Das Betätigungselement 14 besteht aus einer hohlzylindrischen Hülse, die durch den Schaft 36 gebildet wird, und durch eine Betätigungsfläche 16, die sich am hinteren, dem der Düse 32 abgewandten Ende des Betätigungselements 14 befindet. Der Schaft 36 des Betätigungselements ist im Gehäuse 12 axial verschiebbar gelagert. Die Hülse 13 weist die Kulissenbahn 22 der Kulissenführung auf, in die der Gleitstein 21, der am Gehäuse 12 ausgebildet ist, eingreift.

Die Hülse 13 mit der darin gehaltenen Ampulle 17 ist im Gehäuse 12 geführt gehalten und ragt nach hinten in den Schaft 36 des Betätigungselementes 14 hinein. Am hinteren Ende der Hülse 13 ist die Spiralfeder 40 als Kraftspeicher für die Durchführung der Umschaltbetätigung angeordnet.

Die in der Fig. 3 dargestellte Spiralfeder ist dabei so ausgebildet, dass eine Vorspannung der Feder erst dann stattfindet, wenn das Betätigungselement schon einen Teil des Teilbetätigungsweges durchgeführt hat. Dies verringert insbesondere die Mindestbetätigungskraft, die ausgebracht werden muss, damit sichergestellt ist, dass jeder Teilbetätigungshub ununterbrochen als in sich abgeschlossener Hub ausgebracht wird.

Dabei dient die Spiralfeder 40 nicht nur zur Durchführung der Umschaltbetätigung selbst, sondern auch der Überführung des Betätigungselements 14 von seiner am Ende einer Betätigung erreichten Betätigungsendlage in die Betätigungsausgangslage der nachfolgenden Teilbetätigung.

Die Kulissenbahn 22 der Kulissenführung, die an der Hülse 13 ausgebildet ist, besteht aus zwei axial verlaufenden, radial zueinander versetzten Teilabschnitten, die jeweils den Betätigungsweg einer Teilbetätigung definieren, und einem zwischen diesen beiden Abschnitten verlaufenden, die Umschaltbetätigung zwischen den beiden Teilbetätigungen definierenden schrägen Bahnabschnitt. Am Beginn jedes der beiden axial ausgerichteten, die Teilbetätigungen definierenden Abschnitte ist ein die Betätigungsausgangslagen 30a, 30b definierendes Sperrmittel 27 in Form einer Rastnase, die in die Bahn hineinragt, ausgebildet. Die Sperrmittel 27 sind dabei so ausgebildet, dass sie bei Überschreiten einer Mindestbetätigungskraft am Betätigungselement 14 überdrückt werden können. Am hinteren Ende des ersten axialen, die erste Teilbetätigung definierenden Abschnitts der Bahnkurve der Kulissenbahn 22 ist eine Rastkante 33 ausgebildet, die in Betätigungsrichtung von dem in der Kulissenbahn 22 geführten Gleitstein 21 überfahren werden kann, in Gegenrichtung aber sicherstellt, dass der Gleitstein 21 nicht in den axialen Abschnitt der ersten Teilbetätigung zurück gelangt, sondern in dieser Richtung sperrend und den Gleitstein entlang des Verbindungsstücks, das die Schaltbetätigung definiert, bewegt.

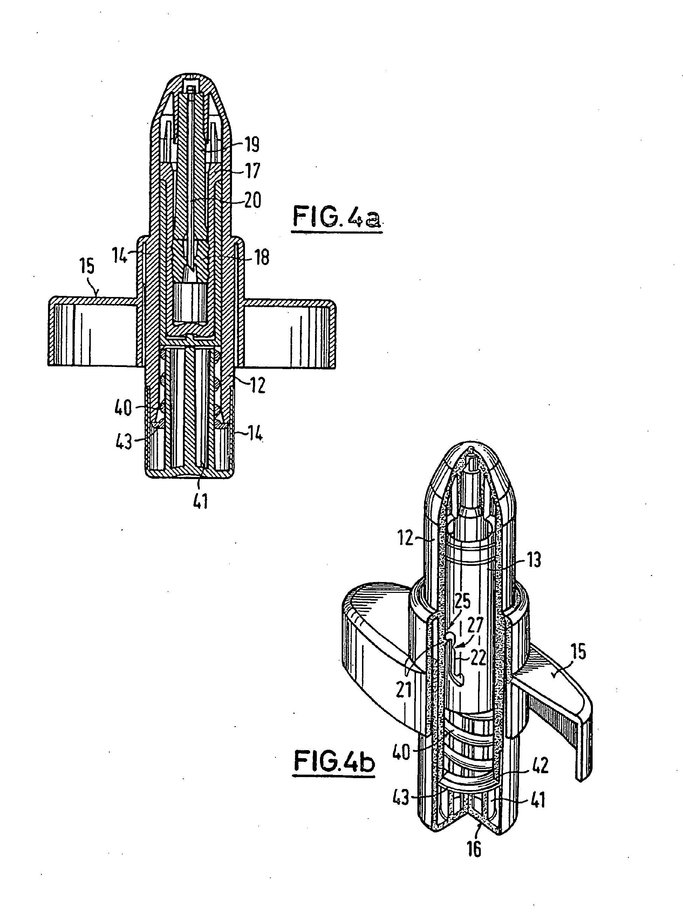

Die Figuren 3a, 3b zeigen die Ausgangsstellung vor der ersten Betätigung des Spenders. Dabei befindet sich der gehäuseseitig ausgebildete Gleitstein 21 in der Betätigungsausgangslage 30a am Beginn des ersten axial verlaufenden Bahnkurvenabschnitts der Kulissenbahn 22, der die erste Teilbetätigung definiert. Diese Lage wird über das Sperrmittel 27 definiert und kann erst durch Überwinden einer Mindestbetätigungskraft, die eine durchgängige, ununterbrochene Betätigung bis in die erste Teilbetätigungs-Endlage, wie sie dann in den Figuren 4a, 4b gezeigt wird, sicherstellt, in die Betätigungsendlage überführt werden. Wird das Betätigungselement 14 an seiner Betätigungsfläche mit mehr als der erforderlichen Mindestkraft betätigt, wobei eine entsprechende Gegenkraft über die Fingerauflage auf das Gehäuse einleitbar ist, so wird der Widerstand des Sperrmittels 27 überwunden und der Gleitstein 21 gleitet in einer geradlinig verlaufenden linearen Bewegung entlang des entsprechenden Abschnitts der Kulissenbahn 22 der Hülse 13. Dies geschieht deshalb, weil das Betätigungselement 14 relativ zum Gehäuse 12 in Richtung auf die Düse 32 hineinbewegt wird, wobei der Stempel 41, der innerhalb des Schaftes 36 des Betätigungselements 14 ausgebildet ist, die Hülse bzw. die in der Hülse angeordnete Ampulle mit einer Kraft beaufschlagt, so dass die Hülse mitsamt der Ampulle 17 in Richtung hin auf die Kanüle 20 im Gehäuse nach vorne geschoben wird. Die Kanüle 20 durchsticht dabei den Stopfen 18, dann gelangt die Kolbenstange 19 in Anlage an den Stopfen 18 und schiebt diesen in der Ampulle 17 nach hinten, wobei das Medienvolumen verringert wird, wodurch Medium durch die Kanüle 20 ausgebracht und in der Düse 32 zerstäubt wird. Während des letzten Viertels dieser ersten Teilbetätigung gelangt die einerseits am Stempel 41 befestigte Spiralfeder in Anlage an die Gehäuseunterkante 42, wozu an der Spiralfeder 40 ein Mitnehmerring 43 ausgebildet ist. Bei der Weiterbewegung des Stempels mit dem Betätigungselement 14 relativ zum Gehäuse 15 wird die Feder vorgespannt. Die Bewegung setzt sich solange fort, bis der Gleitstein 21 das Ende des linearen Abschnittes der der ersten Teilbetätigung entspricht erreicht, wobei kurz vor dem Erreichen des als Umkehrpunkt ausgebildeten ersten Teilbetätigungs-Endlage 25 die Rastkante 33 überwunden wird, die sicherstellt, dass der Gleitstein 21 nicht mehr in den Bahnkurvenabschnitt gelangt, der der ersten Teilbetätigung entspricht.

Diese Situation ist in den Figuren 4a, 4b dargestellt. Die Feder 40 ist vorgespannt. Der Gleitstein 21 ist aufgrund der Rastkante 33 gezwungen, in den Übergangsabschnitt der die Verbindung zwischen den beiden die Teilbetätigungen definierenden Abschnitte der Bahnkurve der Kulisse 22 darstellt, zu gelangen. Dieser Übergangsabschnitt verläuft im wesentlichen quer, geringfügig jedoch nach vorne, wobei die beiden axialen Abschnitte, die die Teilbetätigungen definieren, um mehr als eine Breite der Kulissenbahn zueinander versetzt sind. Durch die bisher erfolgte Betätigung des Betätigungselements 14 wurde der Stopfen 18 in Richtung auf den Grund der Ampulle 17 hineingeschoben und erreicht mit dem Erreichen des Umkehrpunktes durch den Gleitstein 21 in der Kulissenbahn 22 eine definierte Teilbetätigungsendlage 25, die dem Ausbringen einer vorher definierten Menge des Mediums entspricht.

Sobald die Betätigungsfläche 16 losgelassen wird und somit keine Betätigungskraft mehr auf das Betätigungselement 14 einwirkt, erfolgt aufgrund der Vorspannung der Feder 40 die Umschaltbetätigung, bei der die Hülse 13 um eine koaxial zur Betätigungsrichtung während der Teilbetätigungen verlaufende Drehachse relativ zur Ampulle 17 verdreht wird. Wird alternativ die Hülse auch verdrehfest zur Ampulle 17 gehalten, so verdreht sich Hülse 13 samt Ampulle 17 und Betätigungselement 14 zum Gehäuse 12, sobald die Betätigungsfläche 16 losgelassen wird.

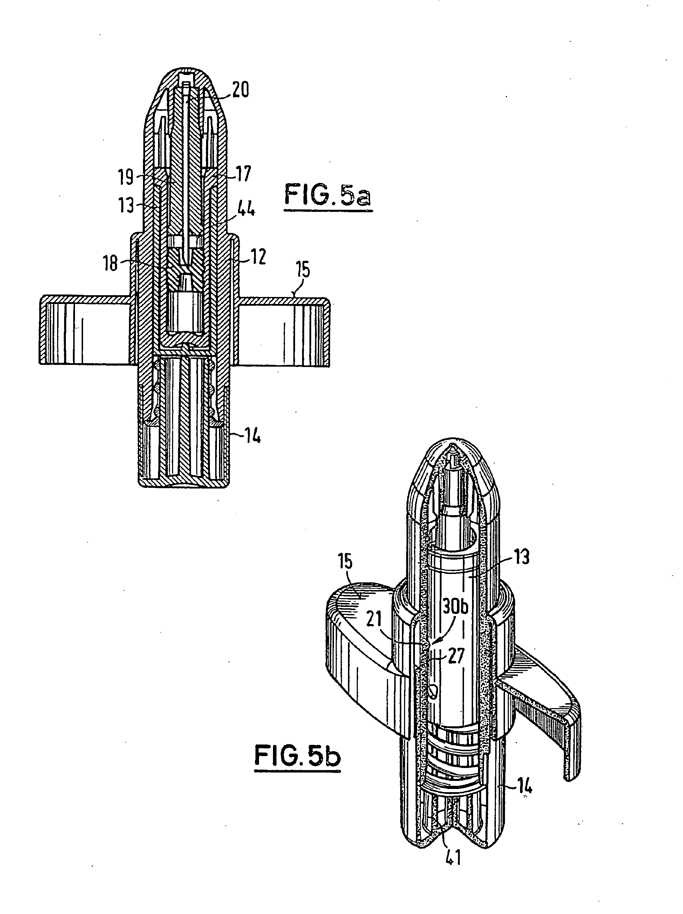

Aufgrund der leicht nach vorne verlaufenden Form der Bahnkurve wird gleichzeitig die Hülse 13 mitsamt der darin befestigten Ampulle 17 um ein geringes Maß im Gehäuse 12 zurückgezogen, so dass zwischen Kolbenstange 19 und dem Stopfen 18 ein geringer Spalt 44 entsteht und die Kanüle 20 teilweise aus dem Stopfen 18 zurückgezogen wird. Diese Lage ist in den Figuren 5a, 5b dargestellt. Sie bilden die Betätigungsausgangslage vor der zweiten Teilbetätigung. Der Gleitstein 21 befindet sich nunmehr in der Betätigungsausgangslage 30b unmittelbar vor dem Sperrmittel 27, das die Mindestbetätigungskraft für die Durchführung einer kontinuierlichen zweiten und letzten Teilbetätigung bis zum Erreichen der Betätigungsendlage sicherstellt. Die Spiralfeder 40 befindet sich dabei weiterhin mit ihrem Mitnehmerring 43 in Anlage an der Gehäuseunterkante 42. Aufgrund dieser Tatsache erfolgt die zweite Teilbetätigung vollständig gegen die Wirkung der Spiralfeder 40. Daher ist eine geringfügig erhöhte Betätigungskraft für die Durchführung der zweiten Teilbetätigung gegenüber der ersten Teilbetätigung erforderlich, was dadurch kompensiert wird, dass die Kanüle 20 den Stopfen nicht mehr vollständig durchstechen muss.

Im Anschluss an die Betätigungsausgangslage 30b erstreckt sich der zweite axial verlaufende Abschnitt der Bahnkurve der Kulissenbahn 22 der Kulissenführung. Sobald das Betätigungselement 14 erneut mit einer Betätigungskraft übersteigt, die die durch das Sperrmittel 27 definierte Mindestmaß übersteigt, wird die Hülse 13 weiter nach vorne geschoben. Die Kanüle 20 durchsticht erneut den Stopfen 18, dieser gelangt nach Überwindung des durch die Spalte 44 vorgegebenen Leerwegs wieder in Anlage an die Kolbenstange 19. Anschließend wird der Stopfen 18 bis zum Grund der Ampulle 17 in die Ampulle hineingeschoben. Die in den Figuren 6a, 6b gezeigte Betätigungsendlage der zweiten und letzten Teilbetätigung des Spenders wird unter Ausbringung der zweiten Teilcharge, die ihrem Volumen nach dem der ersten Teilcharge entspricht, erreicht. Am Ende der Teilbetätigung erreicht der Gleitstein die zweite Teilbetätigungs-Endlage 26 der Kulissenbahn 22. Diese ist vorzugsweise so ausgebildet, dass der Gleitstein 21 in dieser Teilbetätigungsendlage so gehalten wird, dass der Gleitstein allenfalls durch Überwindung einer Rastung wieder in der Kulissenbahn zurückbewegt werden kann.

Auch während der zweiten Teilbetätigung findet die Kraftübertragung vom Betätigungselement 14 auf die Hülse 13 und die Ampulle 17 über den Stempel 41 des Betätigungselements statt.

## Patentansprüche

1. Spender zum ggf. zerstäubten Ausbringen eines insbesondere flüssigen Mediums aus einem Behältnis (17), wobei das Ausbringen des Mediums durch eine Folge von manuellen Teilbetätigungen eines Betätigungselements (14) in definierten Teilchargen erfolgt,
wobei zwischen zwei Teilbetätigungen des Betätigungselements (14) jeweils eine Umschaltbetätigung vorgesehen ist, die Umschaltbetätigung nach Durchführung einer Teilbetätigung selbsttätig erfolgt und Kulissenbahnen (22, 22b) vorgesehen sind, in denen ein Gleitstein (21, 21b) geführt ist, der im Zusammenwirken mit den Kulissenbahnen die Teilbetätigungen voneinander abgrenzt, **dadurch gekennzeichnet, dass** in wenigstens einer der Kulissenbahnen (22, 22b) wenigstens ein Sperrmittel (27) vorgesehen ist, das Teilbetätigungen des Betätigungselements (14) verhindert, soweit nicht eine Mindestbetätigungskraft am Betätigungselement (14) aufgebracht ist, jedoch bei Aufbringung der Mindestbetätigungskraft von wenigstens einem der Gleitsteine (21b) überwindbar ist.

2. Spender nach Anspruch 1, **dadurch gekennzeichnet, dass** die Sperrmittel (27) eine Sollbruchstelle enthalten.

3. Spender nach Anspruch 1 oder 2, **dadurch gekennzeichnet, dass** für jede Teilbetätigung ein Sperrmittel (27) vorgesehen ist.

4. Spender nach einem der Ansprüche 1 bis 3, **dadurch gekennzeichnet, dass** die Sperrmittel einen in die Kulissenbahn ragenden Materialsteg enthalten.

5. Spender nach einem der Ansprüche 1 bis 4, **dadurch gekennzeichnet, dass** die Sperrmittel (27) jeweils vor einer Position in der Kulissenbahn angeordnet sind, die der Gleitstein (21, 21b) vor der Durchführung jeder Teilbetätigung einnimmt.

6. Spender nach einem der Ansprüche 1 bis 5, **dadurch gekennzeichnet, dass** das Behältnis (17) in einem Gehäuse (12) angeordnet ist,
wobei zwischen Gehäuse (12) und Betätigungselement (14) während jeder Teilbetätigung eine Relativbewegung stattfindet und diese Relativbewegung durch eine der Kulissenbahnen geführt ist.

7. Spender nach einem der vorhergehenden Ansprüche, **dadurch gekennzeichnet, dass** die Umschaltbetätigung durch eine Verdrehung zwischen Gleitstein (21, 21b) und Kulissenbahn (22, 22b) erfolgt.

8. Spender nach einem der vorhergehenden Ansprüche, **dadurch gekennzeichnet, dass** eine Hülse (13) zwischen Gehäuse (12) und Behältnis (17) angeordnet ist, wobei die Hülse (12) entweder die wenigstens eine Kulissenbahn (22, 22a, 22b) oder darin geführten Gleitstein (21, 21a, 21b) aufweist, wobei die Hülse lagefest entweder zum Gehäuse (12) oder zum Behältnis (17) angeordnet ist.

9. Spender nach Anspruch 8, **dadurch gekennzeichnet, dass** die Hülse (13) in Richtung der Teilbetätigung lagefest entweder zum Gehäuse (12) oder zum Behältnis (17) und in Richtung der Umschaltbetätigung relativbeweglich dazu angeordnet ist.

10. Spender nach einem der vorstehenden Ansprüche, **dadurch gekennzeichnet, dass** das Behältnis eine einen Schubkolbenzylinder bildende Ampulle (17) ist, die mit einem Stopfen (18) verschlossen ist, wobei am Gehäuse ein Ausbringungskanal ausgebildet ist, der in einer Ausbringungsöffnung (32) mündet, der an seinem der Ausbringungsöffnung abgewandten Seite in einer Kanüle (20) endet, die während der ersten Teilbetätigung den Stopfen (18) durchsticht,
wobei der Stopfen (18) während jeder Teilbetätigung um ein vorgegebenes Maß in Richtung auf den Grund der Ampulle (17) in die Ampulle hineinbewegt wird und ein vorgegebenes Volumenmedium in den Ausbringungskanal verdrängt.

11. Spender nach einem der vorhergehenden Ansprüche, **dadurch gekennzeichnet, dass** während der Teilbetätigung ein Kraftspeicher (23, 40) vorgespannt wird, der nach Abschluss der Teilbetätigung wenigstens die Umschaltbetätigung durchführt.

12. Spender nach Anspruch 11, **dadurch gekennzeichnet, dass** der Kraftspeicher eine Feder, insbesondere eine Spiralfeder (40) ist.

13. Spender nach Anspruch 12, **dadurch gekennzeichnet, dass** der Kraftspeicher ein in eine Kulissenbahn (22a) ragender Federarm (23) ist.

14. Spender nach einem der vorhergehenden Ansprüche, **dadurch gekennzeichnet, dass** die Umschaltbetätigung mit einer axialen Rückbewegung des Behältnisses (17) entgegen der Richtung der Teilbetätigungen erfolgt.

15. Spender nach Anspruch 10 und 14, **dadurch gekennzeichnet, dass** die Rückbewegung die Kanüle (20) teilweise aus dem Stopfen (18) zurückzieht.

## Claims

1. A dispenser for optionally atomised discharge of an in particular liquid medium from a container (17), discharge of the medium proceeding in defined sub-batches by a succession of manual sub-actuations of an actuating element (14), a change-over actuation in each case being provided between two sub-actuations of the actuating element (14), the change-over actuation proceeding automatically after performance of a sub-actuation and slide tracks (22, 22b) being provided, in which is guided a sliding block (21, 21b) which, in cooperation with the slide tracks, delimits the sub-actuations from one another, **characterised in that** at least one barrier means (27) is provided in at least one of the slide tracks (22, 22b), which barrier means prevents sub-actuations of the actuating element (14) unless a minimum actuating force is applied to the actuating element (14) but, on application of the minimum actuating force, can be overcome by at least one of the sliding blocks (21b).

2. A dispenser according to claim 1, **characterised in that** the barrier means (27) include a predetermined breaking point.

3. A dispenser according to claim 1 or claim 2, **characterised in that** a barrier means (27) is provided for each sub-actuation.

4. A dispenser according to any one of claims 1 to 3, **characterised in that** the barrier means include a material web projecting into the slide track.

5. A dispenser according to any one of claims 1 to 4, **characterised in that** the barrier means (27) are in each case arranged in front of a position in the slide track which is assumed by the sliding block (21, 21b) before each sub-actuation is performed.

6. A dispenser according to any one of claims 1 to 5, **characterised in that** the container (17) is arranged in a housing (12), relative motion occurring between the housing (12) and actuating element (14) during each sub-actuation and said relative motion being guided by one of the slide tracks.

7. A dispenser according to any one of the preceding claims, **characterised in that** the change-over actuation proceeds by twisting between sliding block (21, 21b) and slide track (22, 22b).

8. A dispenser according to any one of the preceding claims, **characterised in that** a sleeve (13) is arranged between housing (12) and container (17), the sleeve (12) comprising either the at least one slide track (22, 22a, 22b) or sliding block (21, 21a, 21b) guided therein, the sleeve being arranged in immobile manner relative either to the housing (12) or to the container (17).

9. A dispenser according to claim 8, **characterised in that,** in the direction of the sub-actuation, the sleeve (13) is arranged in immobile manner relative either to the housing (12) or to the container (17) and, in the direction of the change-over actuation, is arranged in a manner capable of relative motion thereto.

10. A dispenser according to any one of the preceding claims, **characterised in that** the container is an ampoule (17) which forms a thrust piston cylinder, which ampoule is sealed with a bung (18), a discharge duct being formed on the housing, which discharge duct leads into a discharge orifice (32) and terminates at its end remote from the discharge orifice in a cannula (20) which punctures the bung (18) during the first sub-actuation, the bung (18) being moved into the ampoule towards the base of the ampoule (17) by a predetermined amount during each sub-actuation and expelling a predetermined volume of medium into the discharge duct.

11. A dispenser according to any one of the preceding claims, **characterised in that** an energy storage means (23, 40) is prestressed during the sub-actuation, which energy storage means carries out at least the change-over actuation after completion of the sub-actuation.

12. A dispenser according to claim 11, **characterised in that** the energy storage means is a spring, in particular a spiral spring (40).

13. A dispenser according to claim 12, **characterised in that** the energy storage means is a spring arm (23) projecting into a slide track (22a).

14. A dispenser according to any one of the preceding claims, **characterised in that** the change-over actuation proceeds with an axial reverse movement of the container (17) contrary to the direction of the sub-actuations.

15. A dispenser according to claim 10 and claim 14, **characterised in that** the reverse movement partially withdraws the cannula (20) out of the bung (18).

## Revendications

1. Distributeur destiné à appliquer, le cas échéant par pulvérisation, un agent en particulier liquide à partir d'un récipient (17), sachant que l'application de l'agent s'effectue par une succession d'actionnements manuels partiels d'un élément de commande (14) selon des doses partielles définies, qu'entre deux actionnements partiels de l'élément de commande (14) est à chaque fois prévu un actionnement de commutation, que l'actionnement de commutation a lieu automatiquement après un actionnement partiel et que sont prévues des coulisses (22, 22b) dans lesquelles est guidé un coulisseau (21, 21b) qui, en interaction avec les coulisses, délimite les actionnements partiels les uns par rapport aux autres, **caractérisé en ce que** dans au moins une des coulisses (22, 22b) est prévu au moins un moyen de blocage (27) qui empêche des actionnements partiels de l'élément de commande (14) tant qu'une force minimale d'actionnement n'est pas appliquée sur l'élément de commande (14), mais qui peut être franchi lors de l'application de la force minimale d'actionnement par au moins un des coulisseaux (21b).

2. Distributeur selon la revendication 1, **caractérisé en ce que** les moyens de blocage (27) sont munis d'un point destiné à la rupture.

3. Distributeur selon la revendication 1 ou 2, **caractérisé en ce qu'**un moyen de blocage (27) est prévu pour chaque actionnement partiel.

4. Distributeur selon l'une des revendications 1 à 3, **caractérisé en ce que** les moyens de blocage sont munis d'une barrette de matière faisant saillie dans la coulisse.

5. Distributeur selon l'une des revendications 1 à 4, **caractérisé en ce que** chaque moyen de blocage (27) est placé en amont d'une position dans la coulisse qui est prise par le coulisseau (21, 21b) avant chaque actionnement partiel.

6. Distributeur selon l'une des revendications 1 à 5, **caractérisé en ce que** le récipient (17) est disposé dans un corps (12), sachant que lors de chaque actionnement partiel a lieu un mouvement relatif entre le corps (12) et l'élément de commande (14), et que ce mouvement relatif est effectué par une des coulisses.

7. Distributeur selon l'une des revendications précédentes, **caractérisé en ce que** l'actionnement de commutation est effectué par un mouvement de rotation entre le coulisseau (21, 21b) et la coulisse (22, 22b).

8. Distributeur selon l'une des revendications précédentes, **caractérisé en ce qu'**une douille (13) est placée entre le corps (12) et le récipient (17), sachant que la douille (12) présente soit l'au moins une coulisse (22, 22a, 22b) soit le coulisseau (21, 21a, 21b) guidé dans elle, sachant que la douille est disposée de manière fixe soit par rapport au corps (12) soit par rapport au récipient (17).

9. Distributeur selon la revendication 8, **caractérisé en ce que** la douille (13) est disposée de manière fixe dans le sens de l'actionnement partiel soit par rapport au corps (12) soit par rapport au récipient (17), et de manière relativement mobile par rapport à lui dans le sens de l'actionnement de commutation.

10. Distributeur selon l'une des revendications précédentes, **caractérisé en ce que** le récipient est une ampoule (17) formant un cylindre à piston-poussoir et fermée avec un bouchon (18), sachant qu'est formé sur le corps un canal d'extraction qui aboutit dans un orifice d'extraction (32), et dont le côté opposé à l'orifice d'extraction se termine par une canule (20) qui perce le bouchon (18) lors du premier actionnement partiel, sachant que pendant chaque actionnement partiel, le bouchon (18) est déplacé dans l'ampoule d'une valeur prédéfinie en direction du fond de l'ampoule (17) et repousse un volume d'agent prédéfini dans le canal d'extraction.

11. Distributeur selon l'une des revendications précédentes, **caractérisé en ce que** pendant l'actionnement partiel est précontraint un accumulateur d'énergie (23, 40) qui effectue au moins l'actionnement de commutation après la fin de l'actionnement partiel.

12. Distributeur selon la revendication 11, **caractérisé en ce que** l'accumulateur d'énergie est un ressort, en particulier un ressort en spirale (40).

13. Distributeur selon la revendication 12, **caractérisé en ce que** l'accumulateur d'énergie est un bras élastique (23) faisant saillie dans une coulisse (22a).

14. Distributeur selon l'une des revendications précédentes, **caractérisé en ce que** l'actionnement de commutation s'effectue avec un mouvement de recul axial du récipient (17) dans le sens opposé à celui de l'actionnement partiel.

15. Distributeur selon les revendications 10 et 14, **caractérisé en ce que** le mouvement de recul retire en partie la canule (20) du bouchon (18).
